# EUROPEAN PATENT APPLICATION

(11) **EP 0 970 621 A1**
(43) Date of publication of application: **12.01.2000**
(21) Application number: 98961389.8
(22) Date of filing: 17.12.1998
(51) Int. Cl.: A23L 2/02, C02F 1/48

(54) **BILBERRY-CONTAINING DRINKS AND PROCESS FOR PRODUCING THE SAME**

(30) Priority: 25.12.1997 JP 35643297
(71) Applicant: Fukuoka, Kazuaki, Tokyo 153-0041 (JP)
(72) Inventor: Fukuoka, Kazuaki, Tokyo 153-0041 (JP)
(74) Representative: Panten, Kirsten
(86) International application number: JP9805713
(87) International publication number: WO9933363

(57) **Abstract**

The present invention provides a beverage containing natural water and bilberry extract, characterized in that bilberry extract predominantly containing anthocyanin salts extracted from bilberry fruit is incorporated thereinto. To the beverage containing the bilberry extract, there may further be incorporated sugar, an aromatic, and vitamins and minerals. The beverage containing the bilberry extract of the present invention is also characterized in that natural water used in the beverage is subjected to a magnetic activation treatment. Alternatively, natural water to be used is not necessarily magnetically activated, and a product beverage, or both natural water to be used and a product beverage may be subjected to the magnetic activation treatment. Therefore, in the present invention, bilberry containing anthocyanin salts effective for relaxing eye strain is incorporated into a beverage whose components have excellent absorption in the human body.

## Description

### Technical Field

The present invention relates to a beverage containing bilberry extract, and, more particularly, to a beverage containing magnetically activated water and bilberry extract which predominantly comprises anthocyanin salts extracted from bilberries.

### Background Art

A variety of beverages containing fruit in forms such as juice or pulp are available on the market. Reasons for their popularity lie not only in that they are tasty and capable of supplying water, but also in that fruit is rich in nutrients such as vitamins.

Bilberries contain nutrients, including many types of vitamins. In addition, clinical tests conducted mainly by public organizations in European countries such as Italy, France, and Germany have confirmed that a variety of anthocyanin salts included in bilberries are effective for relaxing eye strain.

For example, an effect of bilberry anthocyanin salts is disclosed in a number of publications, including "Study on the Effects of Anthocyanin Glucosides on the Nocturnal Vision of Air Traffic Controllers" (extracted from REVUE DE MEDECINE AERONAUTIQUE ET SPATIALE NO. 18); "How Bilberry Anthocyan Salt Glucosides Work on Night Vision Capacity of Pilots" (CONGRESS OF AEROSPACE MEDICAL ASSOCIATION - NEW YORK, APRIL 1965 REVUES CHIBRET NO. 42 AND NO. 44, dedicated to ANTHOCYANIN GLUCOSIDES INSTITUT CHIBRET, CLERMONT-FERRAND); and "A Study of an Effect of a Mixture of Bilberry Anthocyaninoside and Myrtillin on Night Vision" (ASS. OCULISTIQUES 1965 VOL. 196, 1965, P.556-562).

Recently, many people have been suffering from eye strain or like symptoms resulting from working long hours on personal computers and like equipment. Also, there has been demand for beverages that are convenient to drink at a desk in an office and contain neither drugs nor quasi-drugs.

However, heretofore, bilberries, effective for relaxing eye strain, have not been incorporated into beverages.

Bilberries have a vivid purplish-red color, which fades when a considerable period of time elapses from manufacture to sales to consumers. Moreover, bilberries have a drawback of permitting components thereof to precipitate easily. In addition, anthocyanin salts contained in bilberries must be made highly absorbent in the human body. Also, eating bilberries or drinking bilberry juice in a usual manner can hardly attain ingestion of bilberry anthocyanin salts in an amount proven effective, or required, for relaxing eye strain as determined by clinical tests.

Accordingly, an object of the present invention is to provide a beverage which contains anthocyanin salts which are effective for relaxing eye strain in a sufficient amount in the form of bilberry extract, and which is magnetically activated by use of a specific magnetic power generator so that the contained anthocyanin salts are better absorbed in the human body.

Another object of the present invention is to prevent fading of the color of bilberries over a period of time elapsed from production to sales to consumers and precipitation of bilberry components; and also to enhance absorption of anthocyanin salts in the human body.

### Disclosure of the Invention

In view of the foregoing, the present invention provides a beverage containing natural water and bilberry extract, characterized in that bilberry extract predominantly contains anthocyanin salts extracted from bilberries is incorporated thereinto. To the beverage containing bilberry extract, there may further be incorporated sugar, an aromatic, and vitamins and minerals. Preferably, the bilberry extract is incorporated in an amount of 700 g - 1400 g in 1000 L of water.

The beverage containing bilberry extract of the present invention is also characterized in that natural water used in the beverage is subjected to magnetic activation treatment. Alternatively, magnetic activation treatment may be performed on a product beverage, or both natural water to be used and a product beverage, rather than on solely natural water to be used.

The method for producing the beverage containing bilberry extract of the present invention comprises the following two steps: a step of subjecting natural water to magnetic activation treatment, and a step of dissolving, in the natural water subjected to the above-described magnetic activation treatment, bilberry extract predominantly containing anthocyanin salts extracted from bilberries. Alternatively, natural water to be used is not necessarily magnetically activated, and a product beverage or both natural water to be used and a product beverage may be subjected to the magnetic activation treatment.

The beverage according to the present invention has an effect of relaxing eye strain, since bilberries, which contain anthocyanin salts, are incorporated thereinto. Moreover, since water to be used for preparation of the beverage or a product beverage is subjected to magnetic activation treatment, and bilberry extract predominantly containing anthocyanin salts extracted from bilberries is used instead of bilberries *per se*, the color of the beverage does not fade, and the components of the beverage do not precipitate. In addition, anthocyanin salts contained in bilberries is better absorbed in the human body.

### Brief Description of the Drawings

Fig. 1 illustrates a water treatment line of the present invention.
Fig. 2 illustrates production steps for a beverage containing bilberry extract of the present invention.

### Best Mode for Carrying Out the Invention

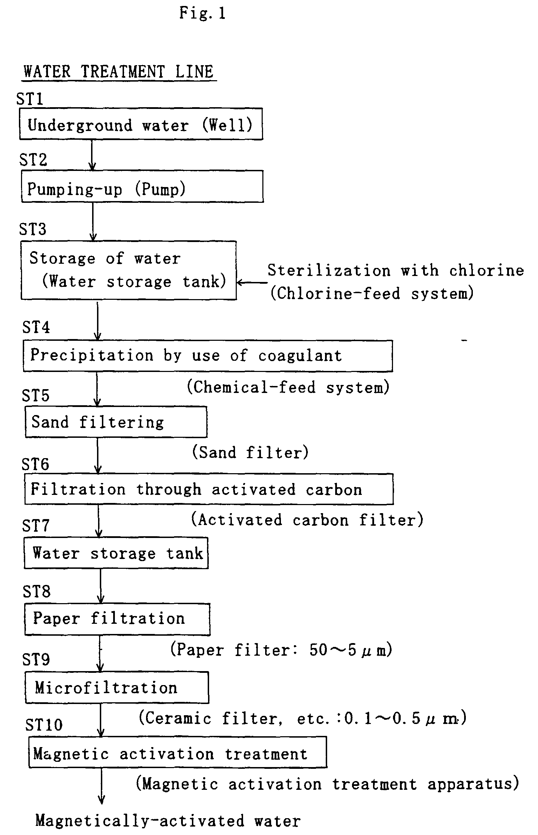

Fig. 1 illustrates a water treatment line for treating water to be used in a beverage containing the bilberry extract of the present invention. Underground water (ST1) is pumped up from a well by use of a pump (ST2), and is then stored in a water storage tank (ST3). Subsequently, the water stored in the tank is sterilized with chlorine injected by a chlorine-feed system. In Step ST4, a coagulant is injected from an chemical-feed system for precipitation, to thereby coagulate impurities through chemical precipitation. In Step ST5, sand filtering is performed by use of a sand filter, followed by activated charcoal filtering employing an activated carbon filter for desalination (ST6). Subsequently, the water is stored in a water storage tank (ST7) and filtered by use of a paper filter of 50-5 µm in Step ST8, followed by microfiltration by use of a ceramic filter of 0.1-0.5 µm or a like filter (ST9).

In the present description, the term "natural water" refers not only to water pumped from a well, but also to water that is sterilized or subjected to removal of its impurities as described above.

At the final step (ST10), the thus-treated water (natural water) is subjected to a magnetic activation treatment employing a magnetic activation system. The magnetic activation treatment is carried out such that a feed-water pipe is sandwiched by a pair of permanent magnets so as to generate a magnetic field most appropriate for activation. Alternatively, the pipe is sandwiched by a plurality of pairs of magnets in accordance with the rate of water flow. The magnetic poles of the pair of magnets may be different from each other; e.g., N pole and S pole, or identical to each other; e.g., S pole and S pole, and in either case an excellent activation effect is realized. The permanent magnets preferably have a high magnetic power, and a magnet obtained by processing a neodymium crystal is suitable. Alternatively, an inexpensive ferrite magnet or a neodymium bond magnet may be used. The feed-water pipe may be made of metals other than ferromagnetic substances such as iron; stainless steels; synthetic resins, etc. in that lines of magnetic force produced from a permanent magnet must be applied onto water in the pipe.

The water molecule (H₂O) has a structure such that each of two hydrogen atoms bonds to the oxygen atom. When water is in a liquid state, water is considered to have a structure such that some water molecules form a cluster by intermolecular force and independent water molecules do not exist. Moreover, it is also known that such water clusters degrade through application of vibration, to reduce in size and thereby activate molecular motion.

In the present invention, a magnetic field is applied to water, to thereby reduce the size of clusters and to activate water. When a magnetic field is applied to water, a variety of ionic substances contained in water; i.e., ions having a variety of structures, move actively in water due to their positive or negative polarity, to thereby reduce the size of clusters. Bilberry anthocyanin salts and minerals are dissolved in water comprising the thus-formed small clusters, to thereby enhance absorbance thereof in the human body and to prevent decoloration and precipitation.

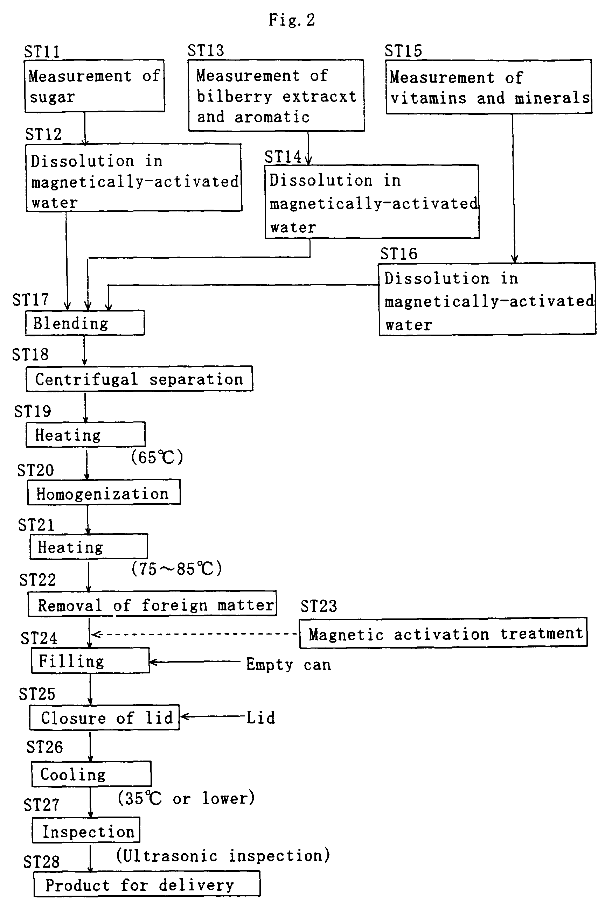

Fig. 2 illustrates production steps for a beverage containing bilberry extract. A sugar ingredient such as fructose-glucose liquid is weighed in Step ST11. In Step ST12, the weighed sugar component is dissolved into water which has been magnetically activated in a manner as described with reference to Fig. 1. Bilberry extract and an aromatic are weighed in Step 13, and are subsequently dissolved in magnetically activated water in a similar manner (ST14).

The bilberry is a perennial shrub which is found in forestland in Europe, Asia, and North America. The bilberry bears small black spherical fruit which include juicy purple pulp. Bilberries have been eaten since ancient times, in that the fruit thereof is juicy, sweet, and aromatic and has many nutrients. Bilberry extract is produced by freezing such fruit; subjecting the frozen fruit to an extraction step; concentrating the resultant extract; purifying; and drying.

In a step (ST15), vitamins and minerals such as citric acid (acidulant), common salt, sodium citrate (seasoning), vitamin C, potassium chloride (salty seasoning), calcium lactate (nutrient), sodium glutamate (nutrient), and magnesium chloride are dissolved in magnetically activated water (ST16).

The above-described ingredients are mixed in appropriate proportions in Step ST17. For example, sugar is added in a content as represented by approximately Bx 6.5 on the Brix scale, and bilberry extract is added in an approximate amount of 700 g - 1400 g based on 1000 L of water. As described above, according to data confirmed in clinical tests, bilberry extract containing approximately 36% anthocyanin salts must be taken in an amount of 100 mg - 200 mg per day so as to develop an effect for relaxing eye strain. If the above dose is to be provided by a beverage contained in a single container; i.e., in a container having a volume of 150 mL, bilberry extract must be incorporated in an amount of approximately 700 g - 1400 g in 1000 L of water.

One example of the thus-prepared beverage containing bilberry extract has the following composition.

To 1000 L of water,

| | |
|---|---|
| (1) Fructose-glucose liquid | 88.9 kg |
| (2) Citric acid (crystalline) | 1.84 kg |
| (3) Common salt | 1.00 kg |
| (4) Sodium citrate | 1.00 kg |
| (5) Vitamin C | 0.50 kg |
| (6) Potassium chloride | 0.37 kg |
| (7) Calcium lactate | 0.15 kg |
| (8) Sodium glutamate | 0.10 kg |
| (9) Magnesium chloride | 0.05 kg |
| (10) Grapefruit essence (aromatic) | 1.00 L |
| (11) Bilberry extract | 1.08 kg |

The thus-prepared beverage containing bilberry extract has a sugar content as represented by 6.5 on the Brix scale, an acid content of 0.20 w/v%, and a content of bilberry extract of 160 mg/150 mL-container.

After preparation (ST17), the beverage is centrifuged (ST18) so as to remove foreign matter, particularly large solids. In Step ST19, the treated beverage is heated at a temperature of, e.g., 65°C, in order to obtain a satisfactory effect in the subsequent step for homogenization. In Step ST20, the product beverage is homogenized to thereby prevent precipitation and is heated again at 75-85°C (ST21). The purpose of heating is to sterilize a container or a cap thereof by heat during a container-filling step. In Step ST22, unnecessary matter which possibly remains is removed by use of a strainer (50-80 mesh), and a vacant can is filled with the treated beverage in Step ST24. Prior to the filling step, the beverage may be magnetically activated (ST23). The magnetic activation treatment is similar to that carried out in a water treatment line as illustrated in Fig. 1. Thus, the treatment will not be further described.

Although the magnetic activation treatment (ST23) is illustrated to be placed just before the filling step in the example described with reference to Fig. 2, the treatment may be carried out at any stage before the filling step. The magnetic activation treatment may be omitted, and there may be carried out only the magnetic activation treatment of water as described above with reference to Fig. 1. Alternatively, the treatment of water may be omitted, and only the prepared beverage may be magnetically treated.

Subsequently, a can is sealed with a cap in Step ST25, and, thereafter, cooled at 35°C or lower (ST26) in order to prevent deterioration of the quality of a product caused by high temperature and to prevent dew formation in a sealed box. In Step 27, ultrasonic inspection is carried out in order to detect air leakage from a can. After a can filled with a high-temperature beverage is cooled, the head space of the can becomes almost vacuum. The principle of the inspection is based on the change of reflection sound to the applied ultrasound corresponding to lowering of vacuum of the head space of the can induced by air leakage. Finally, in Step ST28, the can is filled with the beverage containing bilberry extract of the present invention, to thereby produce a beverage product.

### Industrial Applicability

As described hereinabove, the present invention provides a beverage that contains anthocyanin salts, which are effective for relaxing eye strain, in a sufficient amount in the form of bilberry extract, and that is magnetically activated by use of a specific magnetic power generator so that the contained anthocyanin salts will be better absorbed in the human body.

## Claims

1. A bilberry beverage comprising natural water and a bilberry extract which is prepared from bilberry fruit through extraction and which predominantly contains anthocyanin salts.

2. A bilberry beverage according to claim 1, further comprising sugar, an aromatic, and a vitamin or mineral component.

3. A bilberry beverage according to claim 1, wherein the bilberry extract is incorporated in an amount of 700 g - 1400 g in 1000 L of water.

4. A bilberry beverage according to claim 1, wherein the natural water is subjected to magnetic activation treatment.

5. A method of producing a bilbery beverage, which method comprises a step of subjecting natural water to magnetic activation treatment, and a step of dissolving, in the natural water which has undergone magnetic activation treatment, bilberry extract predominantly containing anthocyanin salts extracted from bilberries.

6. A method of producing a bilberry beverage according to claim 5, which method further comprises a step of subjecting a solution of the bilberry extract to magnetic activation treatment.

7. A method of producing a bilberry beverage, which method comprises a step of dissolving, in natural water, bilberry extract predominantly containing anthocyanin salts extracted from bilberries to thereby obtain a solution, and a step of subjecting the solution of bilberry extract to magnetic activation treatment
